# EUROPEAN PATENT APPLICATION

(11) **EP 1 810 635 A1**
(43) Date of publication of application: **25.07.2007**
(21) Application number: 07000885.9
(22) Date of filing: 17.01.2007
(51) Int. Cl.: A61B 18/16, A61B 18/12

(54) **RF return pad current distribution system**

(30) Priority: 18.01.2006 US 333846
(71) Applicant: Sherwood Services AG, 8200 Schaffhausen (CH)
(72) Inventor: Rick, Kyle R., Boulder Colorado 80303 (US)
(74) Representative: HOFFMANN EITLE

(57) **Abstract**

The present disclosure provides an electrosurgical return pad current detection system for use in monopolar surgery as well as a method of using the same. The detection system includes at least one conductive pad (210) which includes a plurality of conductive elements (220a-220i), wherein each conductive element includes a pad contact impedance (320) and a variable impedance (350). The detection system further includes at least one sensor (400,400a-400f) configured to measure the current levels returning to each conductive element, the current levels being input into a computer algorithm (500). A variable impedance controller (300) is configured to adjust the variable impedance levels based upon output generated by the computer algorithm.

## Description

### BACKGROUND

### Technical Field

The present disclosure is directed to an electrosurgical apparatus and method and, more particularly, is directed to a patient return electrode pad and a method for performing monopolar surgery using the same.

### Background

During electrosurgery, a source or active electrode delivers energy, such as radio frequency energy, from an electrosurgical generator to a patient. A return electrode carries the current back to the electrosurgical generator. In monopolar electrosurgery, the source electrode is typically a hand-held instrument placed by the surgeon at the surgical site and the high current density flow at this electrode creates the desired surgical effect of cutting and/or coagulating tissue. The patient return electrode is placed at a remote site from the source electrode and is typically in the form of a pad adhesively adhered to the patient.

The return electrode typically has a relatively large patient contact surface area to minimize heat concentrated at that patient pad site (i.e., the smaller the surface area, the greater the current density and the greater the intensity of the heat). Hence, the overall area of the return electrode that is adhered to the patient is generally important because it minimizes the chances of current concentrating in any one spot which may cause patient burns. A larger surface contact area is desirable to reduce heat intensity. The size of return electrodes is based on assumptions of the anticipated maximum current during a particular surgical procedure and the duty cycle (i.e., the percentage of time the generator is on) during the procedure. The first types of return electrodes were in the form of large metal plates covered with conductive jelly. Later, adhesive electrodes were developed with a single metal foil covered with conductive jelly or conductive adhesive. However, one problem with these adhesive electrodes was that if a portion peeled from the patient, the contact area of the electrode with the patient decreased, thereby increasing the current density at the adhered portion and, in turn, increasing the heat applied to the tissue. This risked burning the patient in the area under the adhered portion of the return electrode if the tissue was heated beyond the point where normal circulation of blood could cool the skin.

To address this problem, split return electrodes and hardware circuits, generically called Return Electrode Contact Quality Monitors (RECQMs), were developed. These split electrodes consist of two separate conductive foils arranged as two halves of a single return electrode. The hardware circuit uses an AC signal between the two electrode halves to measure the impedance therebetween. This impedance measurement is indicative of how well the return electrode is adhered to the patient since the impedance between the two halves is directly related to the area of patient contact. That is, if the electrode begins to peel from the patient, the impedance increases since the contact area of the electrode decreases. Current RECQMs are designed to sense this change in impedance so that when the percentage increase in impedance exceeds a predetermined value or the measured impedance exceeds a threshold level, the electrosurgical generator is shut down to reduce the chances of burning the patient.

As new surgical procedures continue to be developed that utilize higher current and higher duty cycles, increased heating of tissue under the return electrode may occur. Ideally, each conductive pad would receive substantially the same amount of current, therefore reducing the possibility of a pad site burn. However, this is not always possible due to patient size, incorrect placement of pads, differing tissue consistencies, etc.

### SUMMARY

The present disclosure provides an electrosurgical return electrode current distribution system for use in monopolar surgery. The system includes at least one conductive pad that includes a plurality of conductive elements, wherein the conductive elements include a pad contact impedance and a variable impedance. The system further includes at least one sensor configured to measure the current levels returning to each conductive element, wherein the current levels are input into a computer algorithm. A variable impedance controller is provided that is configured to adjust impedance levels based upon output generated by the computer algorithm.

The present disclosure also provides a method for performing monopolar surgery. The method utilizes the electrosurgical system described above. The method further includes placing the system in contact with a patient, wherein the impedance levels are at some initial value; generating electrosurgical energy via an electrosurgical generator; supplying the electrosurgical energy to the patient via an active electrode; measuring the current returning to each conductive pad; detecting imbalances in current by monitoring the current returning to each conductive pad; and controlling the current entering each pad using a software program and a controller to vary impedances.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is a schematic illustration of a monopolar electrosurgical system according to one embodiment of the present disclosure;
FIG. 2 is a plan view of an electrosurgical return electrode according to one embodiment of the present disclosure, illustrating a conductive pad having a grid of conductive elements of substantially equal sizes;
FIG. 3 is a plan view of an electrosurgical return electrode according to another embodiment of the present disclosure, illustrating a conductive pad having a grid of conductive elements of varying sizes;
FIG. 4 is an enlarged schematic cross-sectional view of a portion of the return electrodes; and
FIG. 5 is an electrical schematic of the RF return pad current distribution system according to one embodiment of the present disclosure.

### DETAILED DESCRIPTION

Embodiments of the presently disclosed RF return pad current distribution system and method of using the same are described herein with reference to the accompanying figures wherein like reference numerals identify similar or identical elements. In the following description, well-known functions or constructions are not described in detail to avoid obscuring the disclosure in unnecessary detail.

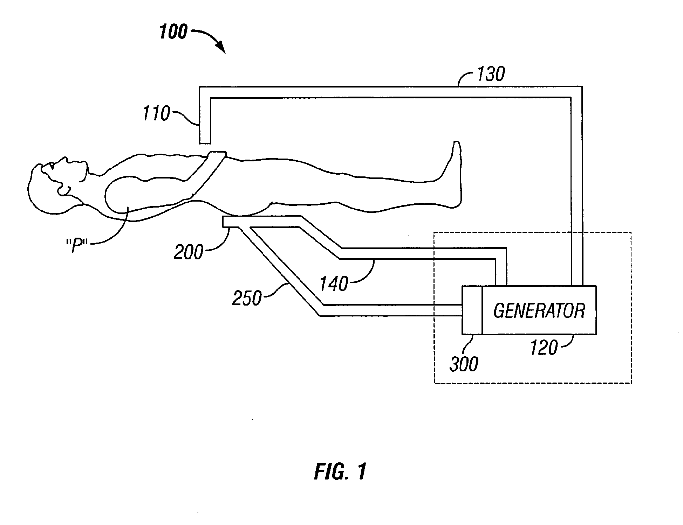

Referring initially to FIG. 1, a schematic illustration of an electrosurgical system 100 is shown. The electrosurgical system 100 generally includes a surgical instrument (e.g., electrosurgical pencil, electrical scalpel or other suitable active electrode) 110, generator 120, return electrode 200, and variable impedance controller 300 coupled to the return electrode 200. In FIG. 1, the return electrode 200 is placed under a patient "P." Electrosurgical energy is supplied to the surgical instrument 110 by the generator 120 via a cable 130 to cut, coagulate, blend, ablate, fuse or vaporize tissue. The return electrode 200 returns energy delivered by the surgical instrument 110 to the patient "P" back to the generator 120 via return path 140.

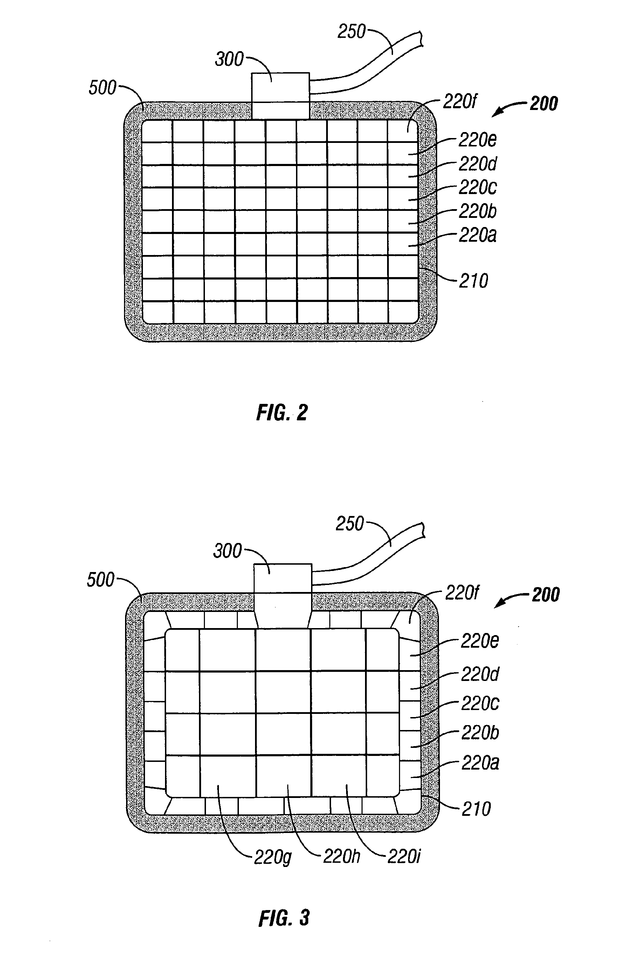

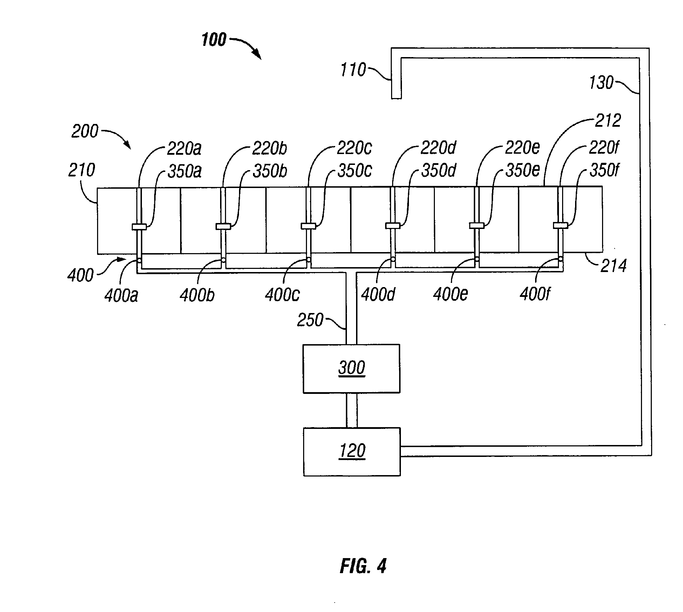

FIGS. 2-5 illustrate various embodiments of the return electrode 200 for use in monopolar electrosurgery. Generally, the return electrode 200 is a conductive pad 210 having a top surface 212 (FIG. 4) and a bottom surface 214 (FIG. 4). The return electrode 200 is operable to receive current during monopolar electrosurgery. While the FIGS. 2-3 depict the return electrode 200 in a general rectangular shape, the return electrode 200 may have any suitable regular or irregular shape such as circular or polygonal. The use of the term "conductive pad" as described herein is not meant to be limiting and may indicate a variety of different pads including, but not limited to, conductive, inductive, or capacitive pads.

As illustrated in FIGS. 2, 3 and 4, the conductive pad 210 includes a plurality of conductive elements (only conductive elements 220a - 220i are labeled for clarity) arranged in a regular or irregular array. Each of the plurality of conductive elements 220 may be equally-sized or differently-sized and may form a grid/array (or may be disposed in any other suitable grid-like arrangement) on the conductive pad 210. The plurality of conductive elements 220a-220f may also be arranged in a suitable spiral or radial orientation (not shown) on the conductive pad 210.

As illustrated in FIG. 4, sensor 400 includes an array of individual sensors (illustrated as 400a-400f, corresponding to conductive elements 220a - 220f, respectively), which are operable to measure the amount of current returning to each pad. The sensor 400 may be coupled to the plurality of conductive elements 220 on the top surface 212, bottom surface 214 of the conductive pad 210 or anywhere therebetween. Moreover, sensor 400 may be located outside of conductive pad 210.

In one arrangement, one sensor 400 is coupled or operatively connected to one of the plurality of conductive elements 220. For example, individual sensor 400a may be coupled to conductive element 220a. Each sensor 400 is connected to the variable impedance controller 300 via a respective cable 250. For example, sensor 400a may be coupled to variable impedance controller 300 via cable 250. In the interest of clarity, each of the cables 250 connected to each sensor 400 is not explicitly illustrated in FIGS. 2 and 3. Furthermore, each conductive element 220a-f is coupled or operatively connected to a respective variable impedance 350a-f, which is, in turn, coupled to variable impedance controller 300. Software program 500 may be located in a variety of locations including, but not limited to, within controller 300 or generator 120.

Sensor 400 is in operative engagement with the return electrode 200 and coupled to the variable impedance controller 300 via a cable 250. The variable impedance controller 300 is coupled to the generator 120 (FIG. 1) and may be affixed to the return electrode 200 (FIGS. 2 and 3), or may be disposed between the return electrode 200 and a generator 120 (FIG. 4).

Generally, the area of the return electrode 200 that is in contact with the patient "P" affects the current density of a signal that heats the patient "P." The smaller the contact area the return electrode 200 has with the patient "P," the greater the current density which directly affects tissue heating at the contact site. Conversely, the greater the contact area of the return electrode 200, the smaller the current density and the less heating of the tissue. Further, the greater the heating of the tissue, the greater the probability of burning the tissue. It is therefore important to either ensure a relatively high amount of contact area between the return electrode 200 and the patient "P," or otherwise maintain a relatively low current density on the return electrode 200.

While there are various methods of maintaining a relatively low current density (including, *inter alia,* the use of electrosurgical return electrode monitors (REMs), such as the one described in commonly-owned U.S. Patent No. 6,565,559, the entire contents of which are hereby incorporated by reference), the present disclosure ensures that return electrode 200 maintains a low current density by sensing and subsequently varying the amount of current returning to each of the plurality of conductive elements 220 of the return electrode 200.

In one embodiment, system 100 operates as follows. Return electrode 200 is placed in substantial contact with a patient's skin. Active electrode 110 is coupled to generator 120, which provides active electrode 110 with RF current. Once active electrode 110 comes into contact with the patient's skin, RF current flows through the body towards return electrode 200. Return electrode 200 includes a conductive pad 210 having a plurality of conductive elements 220a-f, each of which is coupled to a respective sensor 400a-400f. Sensors 400a-f measure the amount of current returning to each conductive element 220a-f. Ideally, substantially the same amount of current will be flowing into each element 220a-f, however, this is unlikely to be the case. Software program 500 receives data from sensors 400a-f and drives variable impedance controller 300. Controller 300 is coupled to variable impedances 350a-f and may increase or decrease the levels of each variable impedance 350 in order to ensure that substantially equal amounts of current are flowing through each conductive element 220a-f.

Variable impedance controller 300 may be located in a number of different areas including within generator 120. Moreover, variable impedance controller 300, sensors 400a-f, conductive pad 210a-f, and software program 500 are all in electrical communication with one another. For example, software program 500 may be located in a variety of different locations including, but not limited to, variable impedance controller 300, sensor 400 (or a common sensing device), or generator 120. Similarly, variable impedance controller 300 may be coupled or operatively connected to software program 500 and may house software program 500. Similarly, as mentioned hereinbefore, generator 120 could contain one, some or all of these elements.

Variable impedance controller 300 may be selected from a number of suitable designs. Some designs may include proportional-integral-derivative control or other forms of digital control. Moreover, variable impedance controller 300 may receive many suitable types of signals including but not limited to control signals, neural network, and fuzzy logic algorithms.

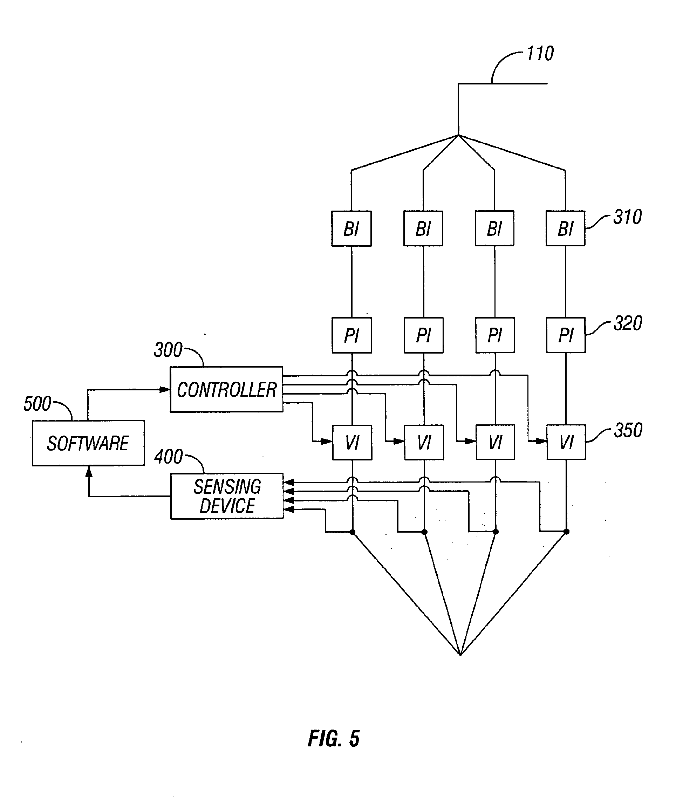

Referring now to FIG. 5, another embodiment of the return pad current distribution system is shown. FIG. 5 shows body impedance (BI) 310, pad contact impedance (PI) 320, and variable impedance(VI) 350 cascaded and interconnected. Body impedance 310 will likely vary depending upon which part of the body is in contact with conductive pad 210. That is, the physiological characteristics may vary significantly from patient to patient and from one sensor to another. Patients may vary in their respective amounts of adipose tissue and certain location sites may be more fatty, hairy, or scarred than another. Electrosurgical system 100 takes into account these factors while providing substantially equal amounts of current through each conductive element 220. Each variable impedance 350 works symbiotically with controller 300, sensor 400, and software 500 to create substantially equal current flow through each conductive element 220a-f.

Variable impedance 350 may take the form of a variable resistor or rheostat. Potentiometers and other suitable devices are also envisioned. Variable impedance 350 is coupled to variable impedance controller 300 and receives directions from controller 300. Variable impedance 350 may be configured in a number of different arrangements. Variable impedance 350 may be attached to conductive pad 210, as shown in FIG. 5, or even housed within conductive pad 210.

The present disclosure also provides a method for performing monopolar surgery. The method may utilize the electrosurgical system 100 described above. The method further includes placing the electrosurgical system 100 in contact with a patient; generating electrosurgical energy via an electrosurgical generator 120; supplying the electrosurgical energy to the patient via an active electrode 110; measuring the current returning to each conductive element 220a-f; detecting imbalances in current by monitoring the current returning to each conductive element 220a-f; and controlling the current entering each element 220a-f using a software program 500 and a controller 300 to vary impedances 350a-f.

The method may further include setting impedances 350a-f to certain predetermined levels using controller 300 in order to direct current towards or away from certain areas.

While several embodiments of the disclosure are shown in the drawings, it is not intended that the disclosure be limited thereto, as it is intended that the disclosure be as broad in scope as the art will allow and that the specification be read likewise. For instance, any mention of devices such as potentiometers and rheostats presupposes that these devices may be digital in nature. Therefore, the above description should not be construed as limiting, but merely as exemplifications of preferred embodiments.

## Claims

1. A return electrode current distribution system, comprising:
at least one conductive pad having a plurality of conductive elements, wherein each conductive element includes a pad contact impedance and a variable impedance;
at least one sensor configured to measure respective current levels returning to each conductive element, the current levels being input into a computer algorithm;
a variable impedance controller, operable to regulate a variable impedance level based upon output generated by the computer algorithm.

2. System according to claim 1, further comprising an electrosurgical generator, operable to regulate an amount of power delivered to the system based upon the current sensed from each conductive pad.

3. System according to claim 2, wherein at least one of the variable impedance controller, sensor, and computer algorithm are housed within the electrosurgical generator.

4. System according to claim 2 or 3, wherein the electrosurgical generator is coupled to at least one of the variable impedance controller, sensor, and computer algorithm and operable to adjust the amount of current provided based upon a control signal from the variable impedance controller.

5. System according to any one of the preceding claims, wherein each conductive element includes a plurality of variable impedances.

6. System according to any one of the preceding claims, wherein the variable impedance controller is selectively adjustable to a predetermined level prior to delivery of current.

7. System according to any one of the preceding claims, wherein the variable impedance is at least one of a rheostat or a potentiometer.

8. System according to any one of the preceding claims, wherein the variable impedance controller utilizes at least one of a proportional-integral-derivative (PID) control and a digital control

9. A return electrode current distribution system, comprising:
a conductive pad having a plurality of conductive elements, wherein each conductive element includes a pad contact impedance and a variable impedance;
at least one sensor configured to measure respective current levels returning to each conductive element, the current levels being input into a computer algorithm;
a variable impedance controller, operable to regulate a variable impedance level based upon output generated by the computer algorithm.

10. The return electrode current distribution system according to claim 9, wherein the conductive pad is either a capacitive or an inductive pad.
